# EUROPEAN PATENT SPECIFICATION

(11) **EP 0 441 976 B1**
(45) Date of publication and mention of the grant of the patent: **17.11.1994**
(21) Application number: 90912945.4
(22) Date of filing: 29.08.1990
(51) Int. Cl.: C07D 241/26

(54) **3,6-DIAMINO-2,5-PYRAZINEDICARBONITRILE AND PRODUCTION THEREOF**
3,6-DIAMINO-2,5-PYRAZINDICARBONITRIL UND IHRE HERSTELLUNG
3,6-DIAMINO-2,5-PYRAZINEDICARBONITRILE ET SA PRODUCTION

(30) Priority: 31.08.1989 JP 223215/89; 13.03.1990 JP 59935/90
(43) Date of publication of application: 21.08.1991
(73) Proprietor: NIPPON SODA CO., LTD., Chiyoda-ku, Tokyo 100 (JP)
(72) Inventor: TAZAKI, Seiji Nippon Soda Co., Ltd., Kurashiki-shi Okayama 711i (JP); YAGIHARA, Tomio Nippon Soda Co., Ltd., Kurashiki-shi Okayama 711Kojimashionasu, (JP); MATSUI, Nobuo Odawara Research Center, Takada, Odawara-shi Kanagawa 250-02 (JP); YANAGISAWA, Atsushi Odawara Research Center, Takada, Odawara-shi Kanagawa 250-02 (JP); KOJIMA, Takakazu Odawara Research Center, Takada, Odawara-shi Kanagawa 250-02 (JP)
(74) Representative: de Bruijn, Leendert C.
(86) International application number: PCT/JP90/01092
(87) International publication number: WO 91/03469

(56) References cited:
- WO-A-88/01264
- FR-A- 2 231 675
- US-A- 3 674 749

## Description

### Technical Field:

This invention relates to 3,6-diamino-2,5-pyrazine-dicarbonitrile (hereafter abbreviated as "the invented compound") which is a new pyrazine derivative having the formula [II] and its preparation methods. 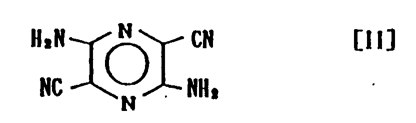
The invented compound is a red crystal and yellowish green fluorescent substance It can be applied as fluorescent dyestuff, light energy conversion material and also as a useful intermediate for synthesizing compounds including such as pteridines.

### Background Art:

Pyrazines are one of the most important classes of nitrogen containing heterocycles. They are widely used for agricultural, pharmaceutical, cosmetic, polymeric chemicals and functional materials. Amino and cyano groups are especially important as the substituents, because of their Potentiality to convert into other functional groups and to introduce various derivatives. Therefore, the pyrazines substituted with both these groups are prospective starting materials.

Since diaminomaleonitrile (hereafter abbreviated as DAMN) and its derivatives are commonly basic starting materials in technical fields, further development of simple synthetic process from DAMN to the useful pyrazines has been expected.

FR-A-2.231.675 relates to a method for Preparing 2,3,5,6-tetracyanopyrazine, 2-amino-3,5,6-tricyanopyrazine and 2,3-dismino-5,6-cyanopyrazine by reacting diamino maleonitrile with an oxidizing agent in the presence of a acidic catalyst.

Pyrazine derivatives, substituted by both amino and cyano groups such as 6-amino-2,3,5-pyrazinetricarbonitrile, 5,6,-di-amino-2,3-pyrazinedicarbonitrile and 3,5-diamino-2,6-pyrazinedicarbonitrile etc., have been synthesized from DAMN or its homologues as the starting materials. (J. Org. Chem., 39, 1235 (1974) and Japanese Patent Application No. Sho 63-75909).

On the other hand, the example of the invented compound having symmetric structure was depicted in USP. 3,674,749 as the starting material for polymers. In that specification, nevertheless, the compound mentioned in the text was quite different from the one shown in the figure. Furthermore, in the description neither physical properties, nor preparation methods and nor examples of usage of "the invented compound" in this specification were present. Never the existence of "the invented compound" has been confirmed.

### Disclosure of Invention:

2,3-diamino-3-(phenylthio)acrylonitrile (hereafter abbreviated as DAAN), a related substance of DAMN, is readily derived from hydrogen cyanide and diphenyl disulfide. The inventors have found the powerful emission of fluorescence in the course of the research on synthesis of new heterocyclic compounds from DAAN, traced the fluorescent compound formed, identified it as "the invented compound" and established its simple synthetic methods by further investigations.

Objects of the invention are "the invented compound" 3.6-diamino-2,5-pyrazinedicarbonitrile which is a new heterocyclic compound, and its preparation methods specified by the reaction of the compounds which are homologues of DAAN, represented by formula [I], wherein R is aryl, alkyl, aralkyl or alkenyl which may be substituted.

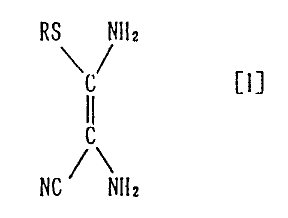

### Best Mode of Carrying Out the Invention:

Description for the invention in details are as follows.

The compounds of general formula [I] as starting materials may be readily synthesized by the methods described in the patent (WO88/01264).

The compounds represented by general formula [I] are allowed to react under the condition of (a) in acidic solutions or (b) in their acid salts solution.

The reaction (a) is generally carried out in organic solvents or in mixed solvents of organic ones and water in the presence of acid or buffer solution for pH adjustment at 0-30°C for one to several hours under atmospheric pressure, with bubbling air if necessary. Hydrocarbons such as benzene, toluene and xylene, nitriles such as acetonitrile, halogenated hydrocarbons such as chloroform and dichloromethane, esters such as ethyl acetate, alcohols such as methanol and ethanol, ketones such as acetone and methyl ethyl ketone, ethers such as tetrahydrofuran, dioxane and 1,2-dimethoxyethane, dimethylformamide and dimethyl sulfoxide as such or mixtures thereof are applicable as the reaction solvents. The acidity of the reaction system, i.e. the pH is advantageously kept at 1-5, particularly desirable at 2-4. Although any acid either an inorganic or organic one may be applied to adjust the pH, it is preferable to use suitable selected buffer solutions. Additionally, the reaction system should be kept under oxidative atmosphere, because the reactions according to the invention are interpreted to be oxidative dimerization condensations. It is recommended that air or oxygen is bubbled into the reaction system, although mere contact with atmospheric oxygen is acceptable.

In the case of reaction (b), that is, using the salts of compounds represented by general formula [I] with acids, polar solvents dissolving the salts, for example, acetonitrile, alcohols, dimethylformamide, dimethyl sulfoxide and their mixtures which are allowable to contain water are selected for the reaction.

The reaction is completed in 2-12 hours, preferably with bubbling air into the system to positively maintain the same oxidative conditions as in the case of reaction (a).

Inorganic acid salts such as hydrochloride, sulfate and nitrate, as well as organic salts such as p-toluenesulfonate, oxalate and picrate can be given as the examples. Furthermore, acids such as trichloroacetic acid and perchloric acid can be utilized in the reaction system although their salts are not likely to be isolated.

In both cases (a) and (b) reaction systems, the proposed compound (the invented compound) can be obtained by usual working up after the completion of reactions.

Identification of the product is confirmed to compare IR, NMR and Mass Spectrometry with known provided isomers of the invented compound.

The invention is further described in more detail by the following examples.

### Example 1.

A solution of 400 mg of DAAN in 45 ml of dimethoxyethane (DME) was added to a solution adjusted to a pH of 3.0 with 150 ml of a buffer solution consisting of 0.1 M citric acid and 0.1 M sodium citrate and 150 ml of water. The reaction mixture was allowed to stand for 5 hours at room temperature. Precipitated red needle crystals were filtered and washed with 3 ml of a solvent mixture of n-hexane and ethyl acetate (3:1).

75 mg of the invented compound was obtained as red needle crystals (postulating one molecule of the invented compound is formed from two molecules of DAAN, the yield was 45%).
Melting point > 280°C.
Mass spectrum: M⁺ = 160.
Absorption spectrum λ max : 223, 266, 458 nm in DME.
Fluorescent spectrum λ em : 538 nm.
(λ ex : 460 nm in DME).
- ¹³C-NMR spectrum:: 149.730,
115.115,
113.251 ppm
(d₆-DMSO).

| HRMASS spectrum | |
|---|---|
| found | 160.0495 |
| calculated (C₆H₄N₆) | 160.0497. |

### Example 2.

Instead of DAAN, 400 mg of 2,3-diamino-3-(4-chloro-phenylthio)-acrylonitrile were reacted in the same manner as Example 1 with bubbling air; 57 mg of the invented compound was obtained as red crystals. (yield: 40.3%).

### Example 3.

A solution of 6253 ml of 0.1 M citric acid and 508 ml of 0.1 M sodium citrate aqueous solution was added to a slurry prepared by mixing 120.0 g of DAAN, 1200 ml of acetonitrile, 285 ml of xylene and 4800 ml of water for adjusting the pH to 3.1. The reaction mixture was stirred under bubbling air at 20°C for 4 hours. Maturing to complete the reaction, red crystals obtained by filtration were washed with 100 ml of water and 150 ml of a solvent mixture of n-hexane and ethyl acetate (1:1). Drying it at 40°C for 4 hours, 38.0 g of the invented compound was obtained as red crystals. (yield: 75.7%).

### Example 4.

70 ml of 1 M sodium acetate aqueous solution and 68 ml of 1 M hydrochloric acid aqueous solution were added to a solution of 6.15 g of DAAN in 76 ml of DME and 457 ml of water for adjusting the pH to 3.7. The reaction was carried out by the same procedures as reported in Example 3. 1.81 g of the invented compound was obtained as red crystals. (yield: 70.3%).

### Example 5.

1 N hydrochloric acid aqueous solution was added to a slurry prepared by mixing 2.05 g of DAAN and 200 ml of chloroform for adjusting the pH to 3.0. The reaction mixture was stirred under bubbling air at 20°C for 4 hours keeping the pH at 3.0 with the occcasional addition of 1 N hydrochloric acid aqueous solution, followed by the same procedures as reported in Example 3. 0.31 g of the invented compound was obtained as red crystals. (yield: 36.2%).

### Example 6.

320 ml of 0.1 M citric acid and 26 ml of 0.1 M sodium citrate aqueous solution were added to a slurry prepared by mixing 6.15 g of DAAN, 76 ml of benzene and 245 ml of water for adjusting the pH to 3.0. The reaction mixture was stirred under bubbling air at 20°C for 4 hours, followed by the same procedures as reported in Example 3. 1.93 g of the invented compound was obtained as red crystals. (yield: 75.1%).

### Example 7.

1 N hydrochloric acid aqueous solution was added to a solution of 2.0 g of DAAN, 42 ml of acetonitrile and 82 ml of water for adjusting the pH to 3.1. The reaction mixture was stirred under bubbling air at 20°C for 4 hours, follwed by the same procedures as reported in Example 3. 0.57 g of the invented compound was obtained as red crystals. (yield: 66.5%).

### Example 8.

107 ml of 0.1 M citric acid and 9 ml of 0.1 M sodium citrate aqueous solution were added to a solution of 2.20 g of 2,3-diamino-3-(p-tolylthio)acrylonitrile in 25 ml of DME and 82 ml of water for adjusting the pH to 3.2. The reaction mixture was stirred under bubbling air at 20°C for 4 hours, followed by the same procedures as reported in Example 3. 0.60 g of the invented compound was obtained as red crystals. (yield: 69.9%).

### Example 9.

113 ml of 0.1 M citric acid and 9.5 ml of 0.1 M sodium citrate aqueous solution were added to a solution of 1.62 g of 2,3-diamino-3-(ethylthio)acrylonitrile in 26 ml of DME and 87 ml of water for adjusting the pH to 3.2. The reaction mixture was stirred under bubbling air at 20°C for 4 hours, followed by the same procedures as reported in Example 3. 0.44 g of the invented compound was obtained as red crystals. (yield: 48.7%).

### Examples 10-15.

The results of syntheses of the invented compound from DAAN as the starting material under various conditions (solvents, buffer solutions, pH etc.) are summarized in Table 1.

**Table 1**

| Example No. | Solvent | Buffer solution | Reacted at 20 °C for 4 hours | |
|---|---|---|---|---|
| | | | pH | Yield(%) |
| 10 | DME | 1 N formic acid + 1 N NaOH | 3.2 | 64.3 |
| 11 | DME | 0.1 N Na citrate + 0.1 N HCl | 2.5 | 61.9 |
| 12 | DME | 0.1 M glycine+0.1 M NaCl+0.1 M HCl | 3.4 | 70.8 |
| 13 | methanol | 0.1 M citric acid+ 0.1 M Na citrate | 3.0 | 70.1 |
| 14 | acetonitrile | 1 N Na acetate + 1 N HCl | 4.0 | 70.4 |
| 15 | xylene | 0.1 N citric acid + 0.1 N Na citrate | 3.1 | 70.5 |

### Example 16.

Air was bubbled through a solution of 0.74 g of DAAN oxalate in 75 ml of acetonitrile for 6 hours for completing the reaction. The reaction mixture was concentrated in evaporator for removing acetonitrile and filtered under adding of water. The precipitate was washed with water and thereafter with a solvent mixture of n-hexane and ethyl acetate (3:2) and dried at 60°C for 2 hours. 0.10 g of the invented compound was obtained as red crystals. (yield: 47.5%).

### Example 17.

A solution of 191.0 g of DAAN and 68.7 g of picric acid in 4800 ml of acetonitrile was stirred at room temperature for 2.5 hours. Formed reddish crystals were washed with 300 ml of a solvent mixture of n-hexane and ethyl acetate (2:1) and dried at 60°C for 3 hours. 56.2 g of the invented compound was obtained as red crystals. (yield: 70.3%).

### Example 18.

0.35 g of trifluoroacetic acid was added to a solution of 2.90 g of DAAN in 50 ml of acetonitrile at room temperature. The reaction mixture was stirred for 3 hours. Precipitated reddish crystals were filtered and washed with a solvent mixture of n-hexane and ethyl acetate (2:1) and dried at 60°C for 2 hours. 0.79 g of the invented compound was obtained as red crystals. (yield: 65.0%).

### Example 19.

A solution of 2.90 g of DAAN and 0.71 g of oxalic acid in a solvent mixture of 75 ml of acetonitrile and 20 ml of water was stirred at room temperature for 3.5 hours. Precipitated reddish crystals were washed with 300 ml of a mixed solvent of n-hexane and ethyl acetate (2:1), and dried at 60°C for 3 hours. 0.55 g of the invented compound was obtained as red crystals. (yield: 45.4%).

### Industrial Applicability:

The invented compound itself is a red pigment and shows powerful yellowish green fluorescence. Therefore, it is possible to apply it as fluorescent dyestuff and light energy conversion material. Additionally, it is a useful intermediate for synthesizing pteridines which are widely utilized in agricultural and pharmaceutical industry.

The preparation methods according to the invention are economically excellent processes. 3,6-diamino-2,5-pyrazinedicarbonitrile, the target compound presented in structural formula [II], is obtained with one step procedure in high yield from the readily available 2,3-diamino-acrylonitrile derivatives.

## Claims

1. 3,6-Diamino-2,5-pyrazinedicarbonitrile.

2. Preparation methods for synthesizing 3,6-diamino-2,5-pyrazinedicarbonitrile from compounds represented by formula [I], 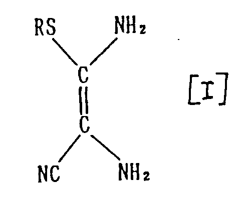 wherein R is aryl, alkyl, aralkyl or alkenyl which may be substituted, as a starting material, under acidic condition in the presence of oxygen.

## Patentansprüche

1. 3,6-Diamino-2,5-pyrazindicarbonitril.

2. Herstellungsverfahren zum Synthetisieren von 3,6-Diamino-2,5-pyrazindicarbonitril aus Verbindungen, angegeben durch die Formel [I] 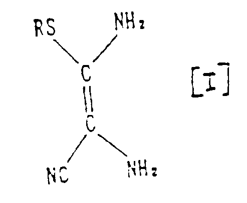 in der R Aryl, Alkyl, Aralkyl oder Alkenyl ist, das substituiert sein kann als Ausgangsmaterial unter sauren Bedingungen in Gegenwart von Sauerstoff.

## Revendications

1. 3,6-Diamino-2,5-pyrazinedicarbonitrile.

2. Procédés de préparation pour synthétiser du 3,6-diamino-2,5-pyrazinedicarbonitrile à partir de composés représentés par la formule [I]. 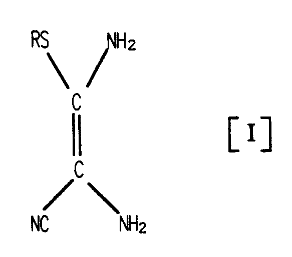 dans laquelle R est un aryle, un alkyle, un aralkyle ou un alcényle qui peuvent être substitués, en tant que matériau de départ, dans des conditions acides en présence d'oxygène.
